# EUROPEAN PATENT APPLICATION

(11) **EP 2 626 014 A2**
(43) Date of publication of application: **14.08.2013**
(21) Application number: 11826146.0
(22) Date of filing: 06.10.2011
(51) Int. Cl.: A61B 17/04

(54) **SURGICAL DEVICE FOR INTERRUPTED SUTURE**

(30) Priority: 07.10.2010 ES 201031491 P
(71) Applicant: Fundacion Para la Investigacion Biomedica Del Hospital Universitario la Paz, 28046 Madrid (ES)
(72) Inventor: CASTELL GÓMEZ, José Tomás, E-28760 Tres Cantos (Madrid) (ES)
(74) Representative: Juncosa Miro, Jaime
(86) International application number: PCT/IB2011/002352
(87) International publication number: WO 2012/046129

(57) **Abstract**

The present invention relates to a surgical device for interrupted suture. It integrates a hollow needle (2) suitable for perforating selectable tissues and allowing the passage there through of a biocompatible T-suture thread (3), with an anchor (4); and a cylindrical rod (7) sized to be introduced and linearly displaced through the inside of the channel of said needle to push the mentioned anchor (4), to expulse it through the distal end of the needle (2). The device further comprises driving means comprising a pusher arranged for pushing the mentioned rod (7) by its proximal end and energy retaining and releasing means associated to the mentioned pusher for - applying a push force thereto and causing, at will, a quick displacement of predetermined length, for pushing the suture once that the end is in a body cavity.

## Description

### Field of the Invention

The present invention belongs to the sector of the surgical medical equipment and more specifically to that of the devices for suture.

The main object of the present invention is a surgical device for interrupted suture which provides a comfortable, quick and safe manner for performing sutures in procedures such as closing the openings (the most used diameters are 5 mm, 10 mm and 12 mm) made on the abdominal wall by means of trocars piercing all the layers thereof, or for fixing meshes to the abdominal wall. Said procedures are applied in surgery with minimally invasive techniques (laparoscopic).

### Background of the Invention

Laparoscopic surgery is a surgical technique which is performed through small incisions using the aid of a video camera allowing the medical team to see the surgical field within the patient and act therein. These techniques are called minimally invasive technique because they prevent the large surgical knife cuts required by open or conventional surgery and they therefore enable a much quicker and comfortable post-operative period.

Their applications are diverse and their field of action broadens each day due to the benefits which have already been mentioned. It can be used in abdominal, articular, gynecological, thoracic surgery, etc. In the case of abdominal laparoscopy, for example, it is necessary to make small incisions where the fine instruments with which the surgeon will perform the operation are introduced through the trocars which are instruments designed to provide the access to the abdominal cavity and form the work channels through which the different surgical instruments are introduced.

These incisions must be surgically sutured to prevent various complications such as hemorrhage or abdominal content herniation. For this purpose, there are various adapted apparatus on the market. However, all of them have in common the fact that the suture is made inside the abdominal cavity and they involve risky and cumbersome handling.

Within this scope, devices comprising a hollow needle and means for introducing a biocompatible suture thread and particularly a T-suture thread which incorporates a stop or blunt rod (rounded ends) called anchor which is adhered to the suture thread, are known.

For such purpose the patent application US 20090082786 describing a medical device and a method for introducing a suture thread with an anchor by means of a hollow needle and pusher system, can be mentioned. Another similar device is described in the application EP 1938760.

There is not a reference in said prior arts relating to the surgical device having means to selectively deliver the thread and anchor into the cavity after traversing the body wall by means of the needle puncture.

### Description of the Invention

The device of this invention is proposed to prevent the difficulties of using those closing systems introduced in the market.

It is a mechanism which comprises a general structure according to a double coaxial sheath. The innermost sheath mounts a biocompatible suture thread integrally incorporating a stop in its end. By means of a plunger, pusher and rod system, once the needle has traversed all the layers of the abdominal wall, the thread is pushed into the body cavity, for example abdominal cavity, such that upon removing the needle the stop anchors the thread in the abdominal wall. Outside this sheath, there is another sheath concentrically arranged which forms the safety mechanism acting in the following manner: The outer sheath or wall of the needle finishes in a sharp end which is the one traversing the different layers of the abdominal wall. When the system perforates all the layers and is in the cavity, the inner sheath or end and blunt portion of the anchor finishing in a blunt end, advances and exceeds the outer sheath. Thus the abdominal viscera are protected from possible tears by the sharp end of the outer sheath.

By performing the same operation on the opposite side of the wound, it is then only necessary to tie the two suture threads to proceed to close the opening.

Therefore, all the operation is performed from outside the organism without internal handlings.

The surgical device for interrupted suture object of the invention comprises for such purpose a loading and deploying mechanism which allows performing interrupted suture procedures which required introducing and fixing a T-suture thread in a selectable tissue in a quick, easy and safe manner. The main advantages of this invention are summarized as follows:
● □The device allows coupling a hollow needle to perforate selectable tissues.
● The device allows mounting a T-suture thread.
● The T-suture thread can be inserted in the device before the puncture.
● The device allows to be loaded, providing a safety position in which:
   o unwanted punctures are prevented since part of the anchor of the T-suture thread is interposed in front of the tip of the hollow needle,
   o making one or several punctures is allowed before delivering the T-suture thread,
   o a transfer indication of the tissue to be sutured is also provided.
● Once the puncture is made, the device allows to be operated, reaching the discharging position in which the T-suture thread is thrown to the other side of the tissue to be sutured.

The surgical device for interrupted suture object of the invention comprises fixing means of the hollow needle, insertion means of the T-suture thread, a main body, a rod, a plunger, an actuator, compression means and restraining means. These elements will be detailed below.

The rod has an elongated cylindrical shape and finishes in a blunt end capable of being introduced into a hollow needle. The plunger has a cylindrical shape and is fixed integral to the rod in one of its bases. The compression means in turn are capable of storing and releasing potential energy through the movement made from and towards the plunger, by means of the intermediation of an actuator.

The main body is a hollow cylindrical structure, in the inside of which are housed the rod, the plunger and the compression means, and in the distal base of which there is an opening through which the rod traverses, whereas in the proximal base the restraining means are coupled as a cover which allow closing the main body, as well as stopping the compression means when the latter are compressed by the operation on the plunger.

The main body has a slot made in its cylindrical surface, such that, together with the intermediation of the actuator, provide the safety position, in which the plunger allows retaining the stored energy in the compression means, and allowing the operator to make several preliminary punctures with the device, the tissue transfer indication being able to be observed (by allowing the backwards movement of the plunger as the needle traverses the muscular wall). Once the suitable puncture has been made, the actuator can be operated to discharge the stored energy, such that the plunger reaches the discharging position and the T-suture thread is thrown to the other side of the tissue to be sutured. Depending on the shape of the slot of the main body, the actuator can be implemented in different ways.

The fixing means of the hollow needle are hollow therein, such that they can be traversed by the rod protruding from the main body and are coupled longitudinally to the hollow needle by the distal side.

The insertion means of the T-suture thread are hollow therein such that they can be traversed by the rod projecting from the main body. These means are attached longitudinally by its distal side to the fixing means of the hollow needle and they are coupled by the proximal side to the main body. These insertion means allow introducing the anchor of the T-suture thread, such that both the anchor and the T-suture thread are transported towards the inside of the hollow needle by means of pushing the rod, when the entire assembly is assembled.

In a preferred embodiment the device can additionally incorporate an attachment element which is hollow therein allowing to be traversed by the rod, and which is coupled longitudinally by its distal side with the insertion means, and by its proximal side to the main body. In this case, a preferred embodiment can also be carried out in which the attachment element in turn incorporates a coupling element and a restriction element associated such that, the glass-shaped coupling element with thread and opening in the proximal base so that it couples the restriction element, can be screwed to the insertion means, whereas the restriction element hollow therein to be traversed by the rod, can be coupled to the coupling element with threading by its distal side, and to the main body by its proximal side, allowing restricting the attachment element to the main body, without rotating the main body or the insertion means when the attachment element is screwed in the insertion means. Thus, the coupling and uncoupling of the assembly is facilitated to load the T-suture thread, keeping the main body and, therefore the rod, independent from the screwing rotations, thus preventing possible knots or cross-links between the T-suture thread and the rod.

In another preferred embodiment, the insertion means of the T-suture thread and the fixing means of the hollow needle form a single element, thus simplifying the structure of the device. This embodiment is interesting for single-use devices which would incorporate in a single assembly the hollow needle and the apparatus with the T-suture thread already loaded for use forming a single use apparatus.

In another preferred embodiment, the plunger and the rod can form a single element, thus simplifying the structure of the device.

Another preferred embodiment in which the restraining means incorporate a pressure regulator such that it allows regulating the energy that can be stored by the compression means could also be contemplated.

The shapes of the main body, the slot made in its surface and the actuator determine the possible preferred embodiments of the device to a large extent. In possible embodiments, the main body can be complete, i.e., of a single piece, whereas in other embodiments, it can be divided into two pieces with rotation relative to one another.

In those embodiments in which the main body is complete, the slot made on the cylindrical surface can have the shape of a step-shaped through groove or also of a through hole, depending on the type of actuator. The step-shaped through groove comprises a distal segment and a proximal segment longitudinally parallel and attached by a transverse segment, such that in the distal part of the proximal segment, there is a bending point to accommodate the safety position. Furthermore, the distal part of the distal segment begins further away than the distal part of the proximal segment, favoring the actuator, and therefore, the plunger, moving inside the main body to the discharging position, thus releasing the energy stored in the compression means, when the actuator is displaced towards the distal segment from the safety position.

In the preferred embodiments in which the main body is complete and the slot is a step-shaped through groove, the actuator is an element integral to the plunger which extends from the cylindrical surface of said plunger. This type of slot allows directly handling the actuator to load the compression means, it allows the actuator to retain said energy in the safety position, it allows the actuator to oscillate along the proximal segment when a puncture is made, showing a transfer indication when the tissue does not offer resistance, and it further allows the actuator to reach the discharging position, in which the stored energy is released after the operator positions the actuator in the distal segment.

In those preferred embodiments in which the main body is complete and the actuator is a stop or pin, the slot can be an opening made in the distal part of the cylindrical surface of the main body, loading the device by pushing the rod against the compression means then being necessary and manufacturing the main body in transparent materials to enable viewing the plunger compressing the compression means until enabling to introduce the actuator in the safety position and enable viewing the plunger oscillating along the main body when a puncture is made, showing the transfer indication when the tissue does not offer resistance, being necessary.

In other preferred embodiments, the main body can be divided into two regular hollow cylindrical parts perpendicular with respect to the axial shaft, such that both parts are coupleable allowing the rotation of one part with respect to the other in relation to the common axial shaft. In those preferred embodiments in which the main body is divided into two assembled elements, called distal and proximal elements, such that there is rotation relative to one another, the actuator can be an element extending from the cylindrical surface of the plunger and, in that case, each of the parts of the main body has a slot which can be a groove in the form of an open segment, with the respective open ends facing one another, such that by rotating an element of the main body with respect to the other, there will be an aligning point of the combined groove. In that aligning point, the passage of the actuator from the distal element to the proximal element is allowed, such that the actuator being in the proximal element, the compression means store energy and if an element of the main body is rotated with respect to the other, the actuator is fixed in the proximal element in the safety position.

These open grooves, in addition to guiding the actuator from one element to another, allow the actuator to oscillate along the open groove of the proximal element when a puncture is made, showing a transfer indication when the tissue does not offer resistance. These grooves can be through grooves, through which the actuator traverses being accessible for handling the mechanism directly, or they can be partial grooves made only in the inner part of the cylindrical surface, through which the actuator is guided although it is not accessible, loading the device by pushing the rod against the compression means then being necessary and manufacturing the main body in transparent materials to enable guiding the actuator to the safety position and to enable viewing the actuator oscillating along the groove when a puncture is made, showing the transfer indication when the tissue does not offer resistance, being necessary.

In another preferred embodiment in which the main body is divided into two parts, the restraining means can be incorporated in the proximal part of the main body.

In other preferred embodiments the transfer indication can be complemented by incorporating a visual and/or sound indicator allowing the operator to know when the tissues to be sutured have been traversed completely.

The basic steps of a procedure for using the device object of the invention which comprises the following steps are described below.
- assembling the device with the hollow needle and the T-suture thread, (this step would not be necessary in single-use devices with the preloaded suture)
- loading the device,
- suitable intermediate puncture of the device with the hollow needle,
- transferring tissue and manual discharge of the device,
- ending the puncture, and
- extracting the device with the hollow needle.

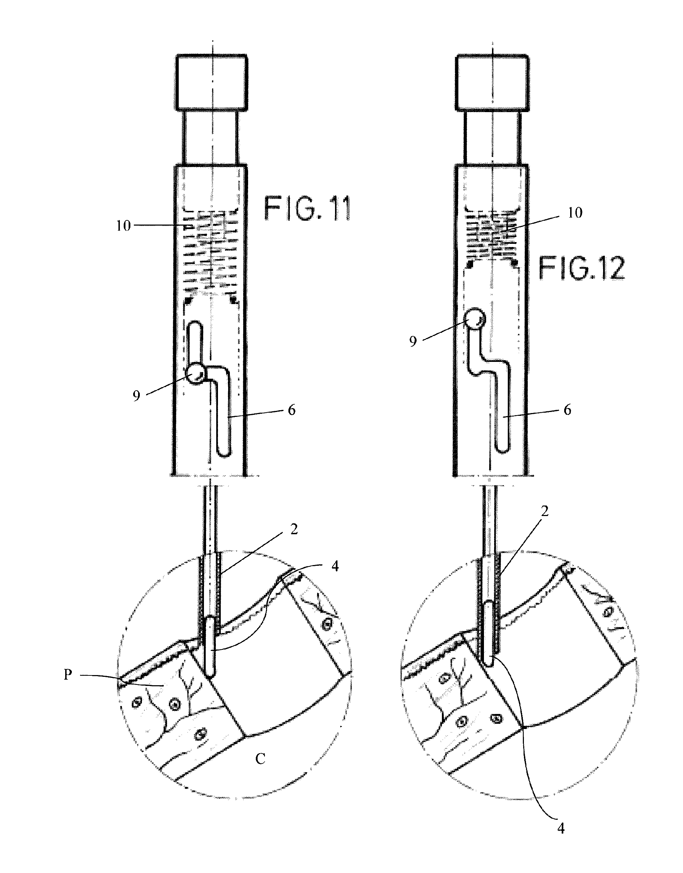

Figures 11 to 15 which will be described below illustrate this operation sequence.

### Brief Description of the Drawings

To complement the description that is being made and for the purpose of aiding to better understand the features of the invention according to a preferred practical embodiment thereof, a set of drawings is attached as an integral part of said description in which the following has been depicted with an illustrative and non-limiting character:
Figure 1 shows a schematic view with a possible embodiment of the device according to the present invention.
Figure 2 shows a schematic view with a possible embodiment of the device assembled according to the present invention.
Figure 3 shows a detailed view of a possible embodiment of the device in which the different elements forming the device are shown in an exploded view.
Figure 4 shows a detailed view of a possible embodiment of the main body and the actuator manipulable from the outside.
Figure 5 to 10 show constructive embodiments of the device.

Finally, Figures 11 to 14 schematically illustrate with an enlarged portion the operation of the surgical device of the invention in an example of its possible use.

### Preferred Embodiment of the Invention

In view of the mentioned drawings and according to the numbering adopted, a preferred embodiment of the invention which comprises the parts and elements indicated and described in detail below can be observed therein.

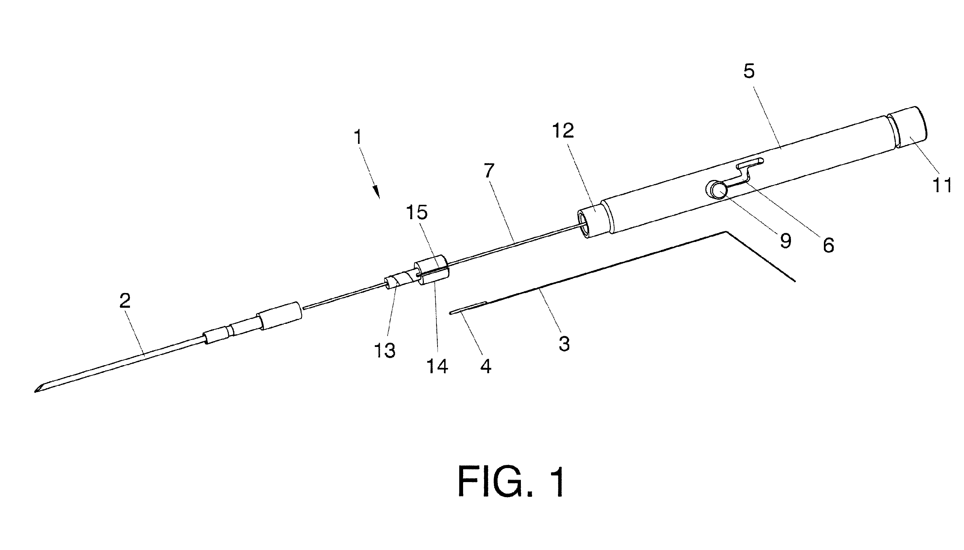

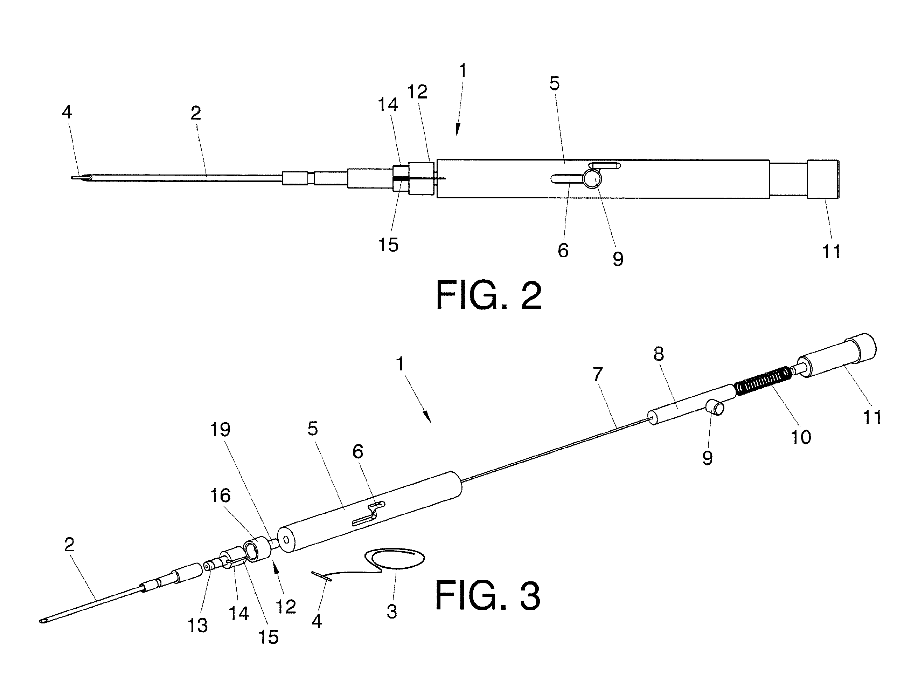

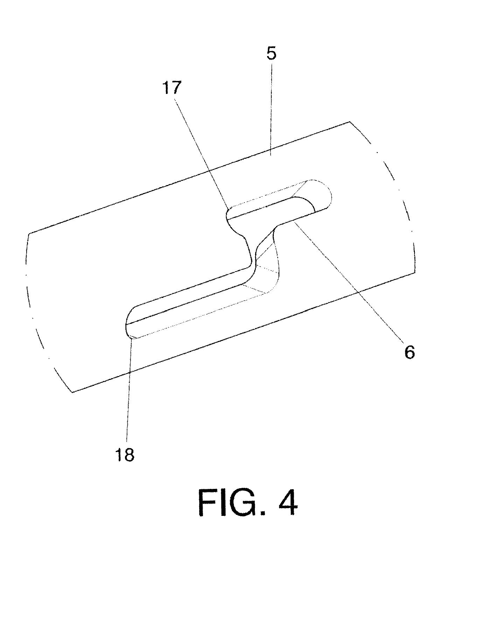

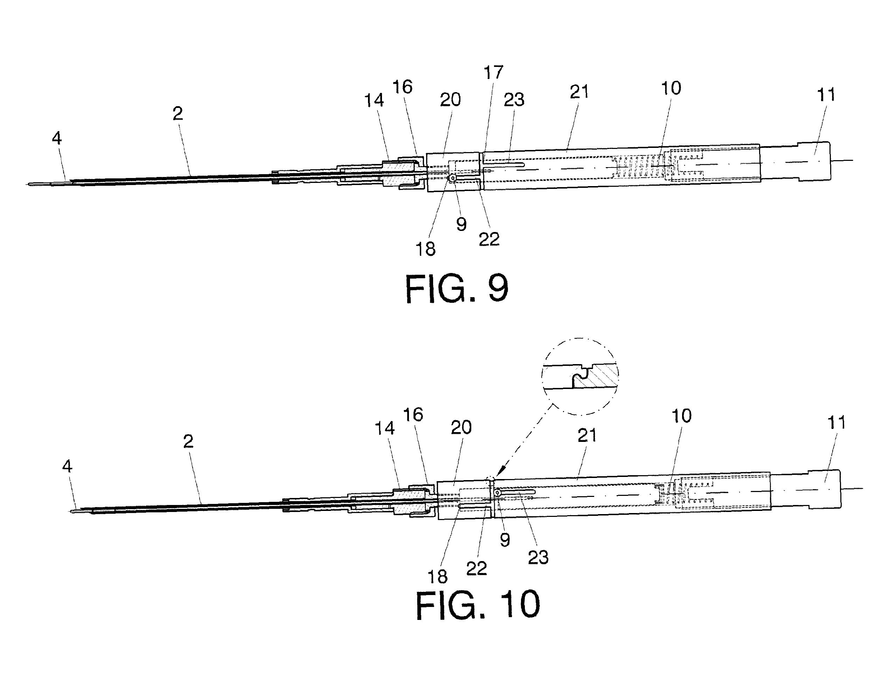

Thus, such as observed in Figures 1 to 3, a possible preferred embodiment of the surgical device (1) for interrupted suture in question manipulable from the proximal part and in the distal part of which there is coupled a hollow needle (2) and a T-suture thread (3) is mounted with the corresponding anchor (4) thereof, essentially comprises the following elements:
- a cylindrical rod (7) with a diameter less than that of an elongated hollow needle (2) finished in a blunt end,
- a plunger (8) integral to the rod (7) by the distal base of said plunger (8),
- compression means (10) for storing and releasing potential energy through the movement made from and towards the plunger (8),
- a main hollow cylindrical body (5) housing therein the rod (7), the plunger (8) and the compression means (10), and in the distal base of which there is an opening through which the rod (7) traverses, and which in turn has a slot (6) with the form of a stepped through groove made in the cylindrical surface of said main body (5) such as shown in Figure 4,
- an actuator (9) intermediating in the slot (6) for retaining the energy stored in the compression means (10), providing the safety position (17) (Figure 9) and allowing making several preliminary punctures observing the tissue transfer indication as a consequence of the movement of the plunger (8), and said actuator (9) to be operated for discharging the stored energy, providing the discharging position (18) (Figure 10) and allowing throwing the T-suture thread (3) to the other side of the tissue to be sutured,
- restraining means (11) coupled as a cover in the proximal base of the main body (5) for closing the main body (5) and stopping the compression means (10) when the latter are compressed by the operation on the plunger (8), said restraining means (11) being configured to be screwed or unscrewed, thus configuring the initial pressure supported by the compression means (10),
- hollow cylindrical-shaped fixing means (13) for fixing the hollow needle (2), allowing the passage of the rod (7) there through, which are coupled longitudinally to the hollow needle (2) by the distal side of said fixing means (13), and
- hollow cylindrical-shaped insertion means (14) for introducing the anchor (4) of the T-suture thread (3), which are attached by the distal part to the fixing means (13) and which have on its surface a longitudinal groove (15) open by the proximal part for inserting the anchor (4) of the T-suture thread (3), and
- an attachment element (12) which is included in this embodiment to facilitate the assembly and which in turn incorporates:
   o a cylindrical threaded glass-shape coupling element (16) to be screwed by its distal side to the insertion means (14), having an opening made in the center of the proximal base allowing the passage of the rod (7), and
   o a hollow cylindrical nut-shaped restriction element (19) allowing the passage of the rod (7), intended to be connected in the hole made in the center of the proximal base of the coupling element (16) and that said hole is opposite to that through which said restriction element (19) is introduced, such that the latter is coupled longitudinally by its proximal side to the main body (5).

In this preferred embodiment, such as observed in Figures 1 to 4, the main body (5) comprises a single piece, the actuator (9) is an element integral to the plunger (8) extending from the cylindrical surface of said plunger (8) and the step-shaped slot (6) is a through groove which allows handling the actuator (9) directly for loading and throwing the compression means (10), comprising a distal segment and a proximal segment longitudinally parallel and attached by a transverse segment such that in the distal part of the proximal segment there is a bending point, for accommodating the safety position (17), allowing the actuator (9) to retain the energy and move along the proximal segment when a puncture is made, showing a transfer indication when the tissue does not offer resistance. Furthermore, the distal part of the distal segment, representing the discharging position (18), begins further away than the distal part of the proximal segment, favoring the actuator (9), and therefore, the plunger (8), moving inside the main body (5), releasing the energy stored in the compression means (10), when the actuator (9) is displaced towards the distal segment from the safety position (18).

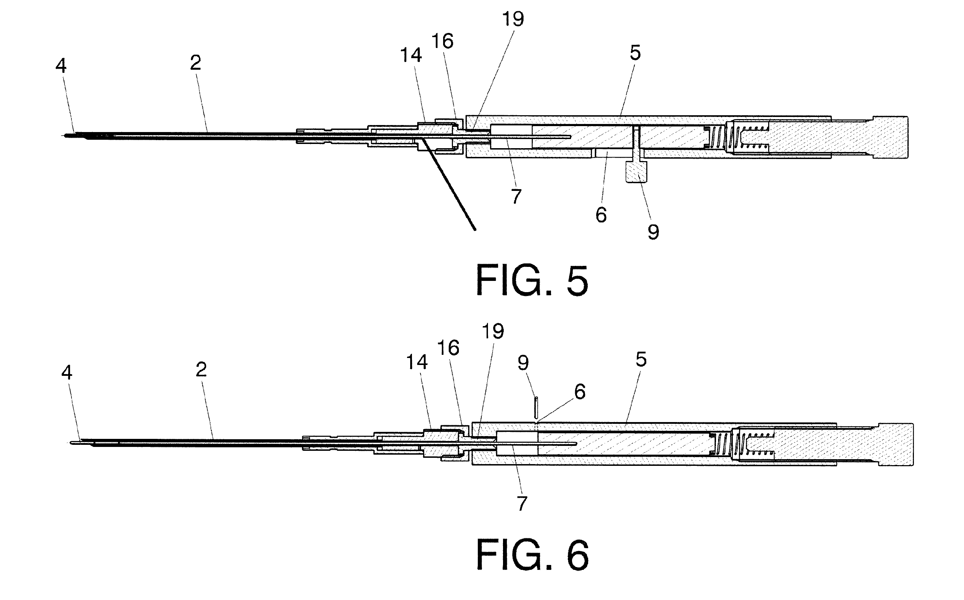

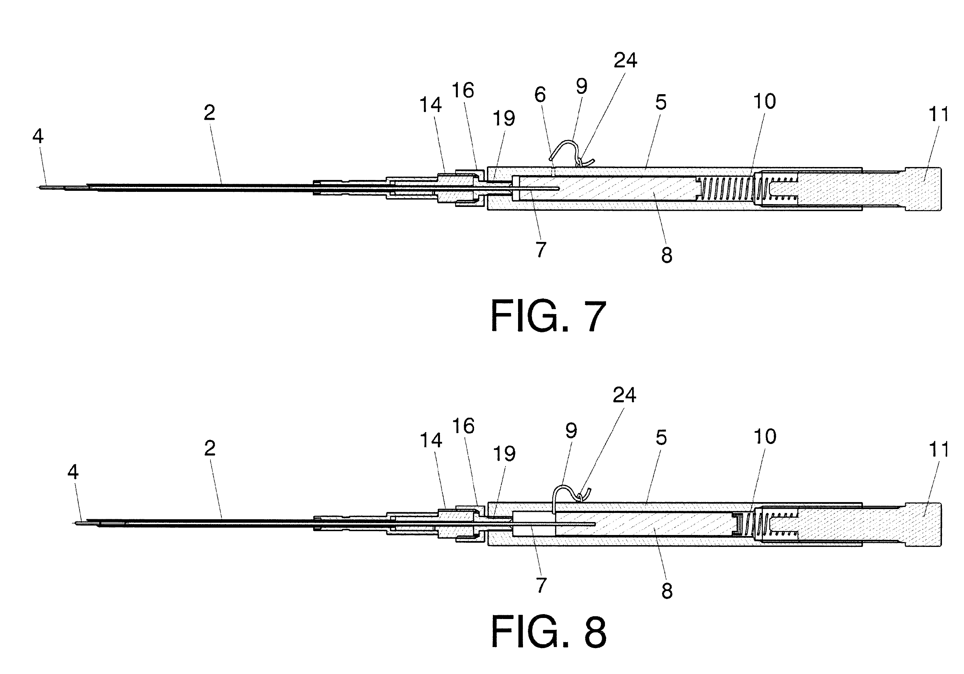

In another preferred embodiment shown in Figures 6 to 8, the main body (5) is also complete but is manufactured in transparent materials, the slot (6) is a through hole made in the distal part of the cylindrical surface of the main body (5), and the actuator (9) can be a free stop or pin such as shown in the Figure 6, or, coupled by means of a lug (24) to the main body (5), as shown in Figures 7 and 8. In this case, loading the device (1) is allowed by pushing the rod (7) against the compression means (10) until being able to introduce the actuator (9) reaching the safety position (17) in which the plunger (8) retains said stored energy, and allows enabling viewing the plunger (8) oscillating along the main body (5) when a puncture is made, showing the transfer indication when the tissue does not offer resistance.

In another preferred embodiment shown in Figures 9 and 10, the main body (5) comprises two cylindrical hollow assembled bodies (20, 21) called distal body (20) and proximal body (21) such that there is rotation relative to one another (20 21) and both (20, 21) having on their cylindrical surface, a distal semi-slot (22) and a proximal semi-slot (23), respectively, and the actuator (9) being an element extending from the cylindrical surface of the plunger (8). These semi-slots (22, 23) can be in the form of a channel open by one of their ends, such that their respective open ends are facing one another. In the aligning point, displacing the actuator (9) from the distal body (20) to the proximal body (21) is allowed, storing energy in the compression means (10) which can be retained in the safety position (17) upon rotating one of the bodies (20, 21) with respect to the other (21, 20). Figure 10 shows in detail, a possible embodiment for assembling the two bodies (20, 21). These semi-slots (22, 23) allow the actuator (9) to oscillate along the proximal semi-slot (23) when a puncture is made, showing a transfer indication when the tissue does not offer resistance. These semi-slots (22, 23) can be through grooves, through which the actuator (9) traverses being accessible to handle the mechanism directly, or they can be partial grooves made only in the inner part of the cylindrical surface of both bodies (20, 21), through which the actuator (9) is guided although it is not accessible, loading the device (1) by pushing the rod (7) against the compression means (10) then being necessary and manufacturing both bodies (20, 21) in transparent materials to enable guiding the actuator (9) to the safety position (17) and to enable viewing the actuator (9) oscillating (going back and forth) along the proximal semi-slot (23) when a puncture is made, thus showing the transfer indication when the tissue does not offer resistance, being necessary.

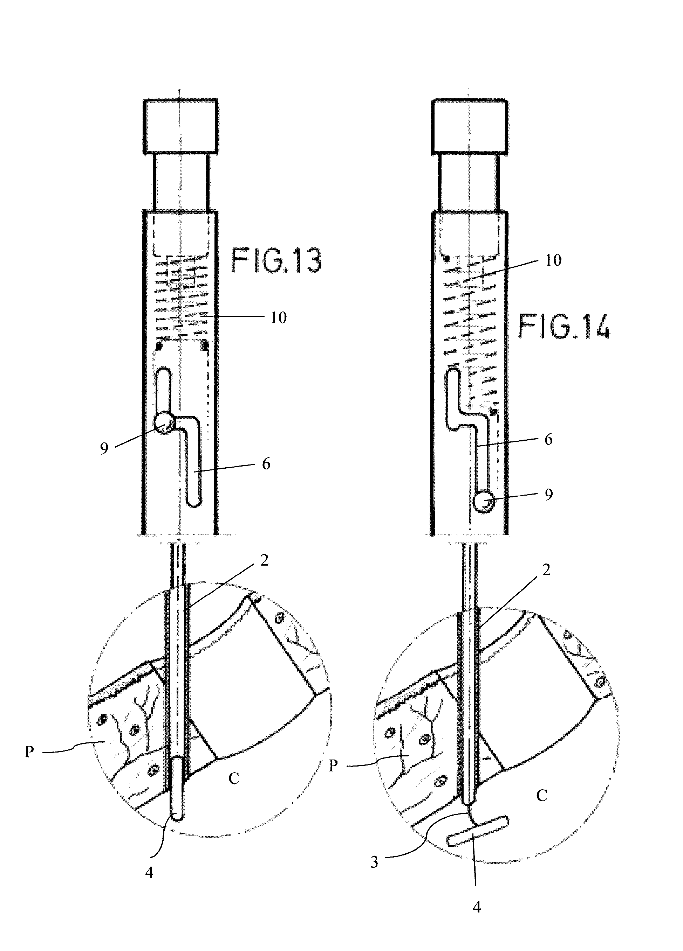

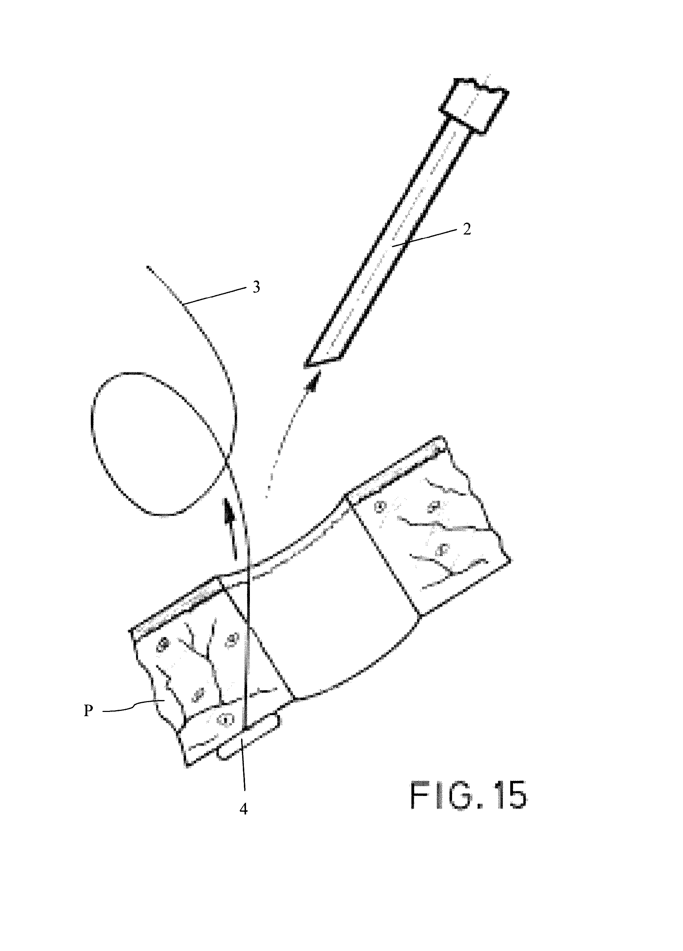

As has been indicated above, Figures 11 to 15 schematically illustrate the operation of the surgical device proposed. The mentioned drawings have illustrated the slot (6) which in cooperation with a spring (10) provides the energy retaining and releasing means which have been described, showing the safety position in Figure 11, the situation of going back the pusher upon transversing a body wall P (Figure 12), the return to the safety position (Figure 13) with the anchor (4) already inside the body cavity C, and finally the throwing of the anchor and thread into the cavity and the removal of the needle (Figure 14). Figure 15 shows the arrangement of the suture already anchored to the inner surface of the cavity C.

## Claims

1. A surgical device (1) for interrupted suture, integrating:
- a hollow needle (2) suitable for perforating selectable tissues and allowing the passage there through of a biocompatible T-suture thread (3) with at least one anchor (4); and
- a comprises two cylindrical hollow assembled bodies rod (7), sized for introducing and linearly displacing the same through the inside of the needle channel to push the mentioned at least one anchor (4), to expulse it through the distal end of the needle (2),
**characterized by** it further integrating driving means comprising:
a pusher arranged for pushing the mentioned rod (7) by its proximal end; and
energy retaining and releasing means associated to the mentioned pusher for applying a push force thereto and selectively causing a quick displacement of predetermined length thereof.

2. The surgical device according to claim 1, **characterized in that** said energy retaining and releasing means comprise:
a plunger (8) integral to the rod (7) by the distal base of said plunger (8);
compression means (10) for storing and releasing potential energy through a movement made from and towards the plunger (8);
an actuator (9) connected to the mentioned plunger which allows linearly displacing the mentioned plunger, acting against said compression means (10); and
a retaining arrangement which allows maintaining said actuator in a first safety position, wherein its displacement and that of the plunger (8) is allowed by loading the compression means and in returning to said first safety position, the linear movement of which provides information on the tissue transfer, and has a second position, wherein the actuator (9) positioned therein is released and the plunger (8) is pushed freely by the mentioned compression means and wherein the suture thread (3) is thrown to the other side of the tissue to be sutured.

3. The surgical device according to claim 2, **characterized in that** it comprises a main hollow cylindrical body (5) housing therein the rod (7), the plunger (8) and the compression means (10), and in the distal base of which there is an opening through which the rod (7) passes, and which in turn has a slot (6) made in the cylindrical surface of said main body (5) in which an actuator (9) can be displaced and which provides the mentioned retaining arrangement.

4. The surgical device according to claim 3, **characterized in that** the actuator (9) is an element integral to the plunger (8), extending from the cylindrical surface of said plunger (8) and **in that** the slot (6) is a step like through groove, comprising a distal segment (6a) and a proximal segment (6b) longitudinally parallel and attached by a transverse segment (6c).

5. The surgical device (1) according to claim 3, **characterized in that** it additionally comprises:
hollow cylindrical-shaped fixing means (13) coupled in its distal side to the hollow needle (2), allowing the passage of the rod (7) there through, and
hollow cylindrical-shaped insertion means (14) for inserting the anchor (4) of the suture thread (3), allowing the passage of the rod (7) there through, being longitudinally attached by its distal side to the fixing means (13) and coupled by the proximal side to the main body (5).

6. The surgical device (1) according to claim 5, **characterized in that** the insertion means (14) incorporate in the cylindrical surface thereof a longitudinal groove (15) open by the proximal end for inserting the anchor (4) of the suture thread (3).

7. The surgical device (1) according to claim 6, **characterized in that** the fixing means (13) and the insertion means (14) form a single element.

8. The surgical device (1) according to claim 2, **characterized in that** the rod (7) and plunger (8) form a single element.

9. The surgical device (1) according to claim 3, **characterized in that** it additionally comprises an attachment element (12) to facilitate the assembly and which is hollow therein allowing to be traversed by the rod (7), and which is coupled longitudinally by its distal side with the insertion means (14) and, by its proximal side with the main body (5).

10. The surgical device (1) according to claim 9, **characterized in that** the attachment element (12) in turn comprises a coupling element (16) and a restriction element (19), wherein the coupling element (16) has an opening made in the center of its proximal base allowing the passage of the rod (7), being screwed by its distal side to the insertion means (14) and, such that, the restriction element (19) allows the passage of the rod (7) and is coupled by its proximal side to the main body (5) such that it allows the rotating movement of said coupling element (16) with respect to the main body (5).

11. The surgical device (1) according to claim 5, **characterized in that** the main body (5) is manufactured in transparent material which allows viewing the plunger (8) oscillating along the main body (5) when a puncture is made showing the transfer indication when the tissue does not offer resistance, the actuator (9) is a pin and the slot (6) is a through hole made in the distal part of the cylindrical surface of the main body (5).

12. The surgical device (1) according to claim 11, **characterized in that** the actuator (9) is articulated with respect to the main body (5) with the intermediation of a lug (24).

13. The surgical device (1) according to claim 5, **characterized in that** the actuator (9) extends from the cylindrical surface of the plunger (8) and **in that** the main body (5) comprises two cylindrical hollow assembled bodies (20, 21) called distal body (20) and proximal body (21), provided with rotation relative to one another, wherein the slot (6) is formed by a distal semi-slot (22) and a proximal semi-slot (23) defined respectively in each of the bodies (20, 21), said semi-slots being in the form of channels open by one of their ends, such that their respective open ends are facing one another, both semi-slots (22, 23) being able to be aligned such that, on one hand, they allow the passage of the actuator (9) from the distal body (20) to the proximal body (21) to store energy in the compression means (10) positioning the plunger (8) in the safety position (17) and retaining said energy upon rotating one of the bodies (20, 21) with respect to the other (21, 20), and, on another hand, the passage of the actuator (9) from the proximal body (21) to the distal body (20) is allowed to release the stored energy, displacing the plunger (8) to the discharging position (18).

14. The surgical device (1) according to claim 13, **characterized in that** the semi-slots (22, 23) are through grooves through which the actuator (9) traverses.

15. The surgical device (1) according to claim 12, **characterized in that** the two bodies (20, 21) are manufactured in transparent material, and **in that** the semi- slots (22, 23) are partial grooves made in the inner part of the cylindrical surface of both bodies (20, 21), through which the actuator (9) is guided although it is not accessible.
